# DEMANDE DE BREVET EUROPEEN

(11) **EP 3 791 722 A1**
(43) Date de publication de la demande: **17.03.2021**
(21) Numéro de dépôt: 19306102.5
(22) Date de dépôt: 13.09.2019
(51) Int. Cl.: A01N 37/02, A01N 25/18

(54) **COMPOSITION REPULSIVE ET UTILISATION**

(71) Demandeur: Université de Bourgogne, 21000 Dijon (FR); Institut national de recherche pour l'agriculture, l'alimentation et l'environnement, 75007 Paris (FR); Centre National de la Recherche Scientifique, 75016 Paris (FR)
(72) Inventeur: BERTHELOT-GROSJEAN, Martine, 21270 SAINT-SAUVEUR (FR); GROSJEAN, Yaël, 21270 SAINT-SAUVEUR (FR); MANIERE, Gérard, 21490 BELLEFOND (FR)
(74) Mandataire: Novagraaf Technologies

(57) **Abrégé**

La présente invention se rapporte à l'utilisation d'au moins un acide gras choisi dans le groupe comprenant l'acide propionique, l'acide butyrique et/ou un dérivé de ceux-ci comme principe actif répulsif et/ou pour contrôler la reproduction de brachycères, et à l'utilisation d'une composition, en particulier phytosanitaire, comprenant au moins un acide gras choisi dans le groupe comprenant l'acide propionique, l'acide butyrique et/ou un dérivé de ceux-ci comme principe actif répulsif et/ou pour contrôler la reproduction de brachycères.

La présente invention trouve notamment une application dans le domaine agricole, vétérinaire, phytosanitaire.

## Description

### Domaine technique

La présente invention se rapporte à l'utilisation d'au moins un acide gras choisi dans le groupe comprenant l'acide propionique, l'acide butyrique et/ou un dérivé de ceux-ci comme principe actif répulsif et/ou pour contrôler la reproduction de brachycères.

La présente invention se rapporte également à l'utilisation d'une composition, en particulier phytosanitaire, comprenant au moins un acide gras choisi dans le groupe comprenant l'acide propionique, l'acide butyrique et/ou un dérivé de ceux-ci comme principe actif répulsif et/ou pour contrôler la reproduction de brachycères.

La présente invention trouve notamment une application dans le domaine agricole, vétérinaire, phytosanitaire.

Dans la description ci-dessous, les références entre crochets ([ ]) renvoient à la liste des références présentée à la fin du texte.

### Etat de la technique

De nombreux insectes dans le domaine agricole sont considérés comme des insectes ravageurs. Il s'agit en particulier d'insectes provoquant et/ou à l'origine de dégâts et/ou d'altération de culture.

Toutes les cultures agricoles, par exemple céréalières, fruitières, maraichères peuvent être affectées par des insectes ravageurs. Les insectes ravageurs sont, de manière générale, spécifiques d'un type de culture et/ou de plante et peuvent affecter différemment les cultures et/ou les plantes. En d'autres termes, il est fréquent que les insectes ravageurs soient spécifiques d'une culture/plante et que l'effet sur la culture/plante soit spécifique.

Par exemple, les insectes ravageurs peuvent avoir un effet sur le fruit de la plante, par exemple de par son alimentation, et/ou affecter la « santé » de la plante, par exemple en véhiculant des pathologies, par exemple des virus, des champignons, etc, susceptibles d'altérer la croissance et/ou la production de fruits/fleurs etc, de la plante/culture.

Toutes les parties de la plante/culture sont susceptibles d'être affectées par des insectes ravageurs. Par exemple les insectes ravageurs peuvent altérer les feuilles, les tiges, les bourgeons, l'écorce, le bois, les racines, les fleurs, les fruits, les graines, à tous les stades de leur développement. En outre, les insectes ravageurs peuvent également affecter les produits/fruits et/ou tout autre élément issu de récoltes.

Il existe donc un réel besoin dans l'état de la technique de protéger et/ou de prévenir l'attaque et/ou l'altération de plantes/cultures par des insectes ravageurs. Il existe également un réel besoin de protéger et/ou de prévenir l'attaque et/ou l'altération des récoltes par des insectes ravageurs.

Afin de préserver les plantes/cultures des insectes ravageurs, des procédés et/ou produits sont connus et utilisés. Par exemple, l'alternance d'une année sur l'autre et/ou des parcelles agricoles des plantes cultivées et/ou d'une sélection de plantes particulières est utilisée afin de réduire les effets d'insectes ravageurs. Toutefois, la protection et/ou les effets sont limités. En outre, la sélection de plantes résistantes peut amener à un appauvrissement génétique des plantes indigènes et/ou à une diminution de la biodiversité.

Des procédés mécaniques sont également utilisés. Des procédés chimiques sont également utilisés, notamment l'utilisation de pesticides de synthèses et/ou chimiques, de compositions répulsives etc. Toutefois, ces procédés ont un effet « non sélectif » ciblant indifféremment tous les insectes et peuvent donc avoir des effets néfastes sur des insectes auxiliaires et/ou essentiels tels que les abeilles et/ou les bourdons. En outre, les procédés chimiques sont actuellement controversés, notamment car ils sont susceptibles d'affecter la santé humaine et/ou être à l'origine de pollution des sols, de cours d'eau, etc. De plus, les insecticides utilisés peuvent être absorbés par les plantes. Ainsi, les plantes et/ou les cultures produites peuvent présenter des concentrations de ces composés non compatibles avec une consommation animale et/ou humaine.

Il existe donc un réel besoin de trouver un nouveau moyen et/ou une nouvelle composition palliant ces défauts, inconvénients et obstacles de l'art antérieur, en particulier un procédé et/ou une composition permettant de préserver les plantes et/ou les cultures des insectes ravageurs, tout en conservant et/ou préservant l'écosystème, la biodiversité et/ou la santé humaine et/ou la santé animale.

### Description de l'invention

La présente invention a précisément pour but de répondre à ces besoins et inconvénients en fournissant au moins un acide gras choisi dans le groupe comprenant l'acide propionique, l'acide butyrique et/ou un dérivé de ceux-ci pour utilisation comme principe actif répulsif et/ou pour contrôler la reproduction de brachycères.

En effet, les inventeurs sont les premiers à avoir mis en évidence, de manière surprenante et inattendue, que l'utilisation selon l'invention, en particulier l'utilisation des acides gras selon l'invention, a un effet sur le comportement d'insectes ravageurs, notamment par la perception olfactive de ces molécules.

En particulier, les inventeurs ont démontré de manière surprenante que l'utilisation des acides gras selon l'invention a un effet répulsif, permet de diminuer voire d'abolir la parade des insectes et peut induire une anesthésie des insectes.

Les inventeurs ont également démontré de manière surprenante que la présente invention, par exemple de par la diminution de la parade, permet avantageusement de réduire et/ou prévenir la prolifération d'insectes ravageurs.

Les inventeurs ont également démontré de manière surprenante que la présente invention permet avantageusement de réduire et/ou prévenir la reproduction et/ou copulation d'insectes ravageurs.

En outre, les inventeurs ont démontré de manière surprenante que la présente invention a un effet sur l'olfaction d'insectes ravageurs, et permet avantageusement de diminuer et/ou de supprimer la détection par des insectes ravageurs de signaux olfactifs attracteurs, par exemple induisant et/ou susceptible de stimuler la parade précédant l'accouplement des insectes. En d'autres termes, les inventeurs ont démontré de manière surprenante que la présente invention permet avantageusement d'altérer la détection par des insectes ravageurs de signaux olfactifs attractifs, par exemple entre mâle et femelle.

De plus, l'invention utilise des d'acides gras et/ou dérivés de ceux-ci qui sont des composés et/ou molécules non toxiques pour l'environnement, respectueuses des écosystèmes et sans effets néfastes connus sur la santé humaine et animale.

La présente invention a pour objet l'utilisation d'au moins un acide gras choisi dans le groupe comprenant l'acide propionique, l'acide butyrique et/ou un dérivé de ceux-ci comme principe actif répulsif et/ou pour contrôler la reproduction de brachycères.

La présente invention a également pour objet l'utilisation d'au moins deux acides gras choisis dans le groupe comprenant l'acide propionique, l'acide butyrique et/ou un dérivé de ceux-ci comme principes actifs répulsifs et/ou pour contrôler la reproduction de brachycères.

Dans la présente le terme « dérivé » désigne un composé chimique ou une molécule fabriquée à partir d'un composé parent par une ou plusieurs réactions chimiques.

Dans la présente invention, le terme «sel» désigne des sels appropriés pour une utilisation pharmaceutique humaine ou vétérinaire sans toxicité, irritation, réponse allergique ou autre effet délétère impropre à une utilisation médicamenteuse et présentent un rapport bénéfice / risque raisonnable.

Dans la présente invention, le terme «sel» désigne également des sels appropriés pour une utilisation phytosanitaire sans toxicité, irritation, réponse allergique ou autre effet délétère impropre à une utilisation, par exemple agricole.

Dans la présente, le terme « répulsif » signifie un éloignement et/ou un repoussement des brachycères de plantes et/ou de végétaux et/ou de fruits et/ou de tout ou partie de plantes et/ou de végétaux et/ou de mammifères. Le terme « répulsif » signifie également une inhibition de l'attractivité des brachycères pour des plantes et/ou des végétaux et/ou des fruits et/ou tout ou partie de plantes et/ou de végétaux et/ou de mammifères.

Dans la présente, le terme « contrôle de la reproduction » signifie une inhibition et/ou une stabilisation de la reproduction de brachycères notamment par inhibition de la parade et/ou une diminution de la parade, et/ou une inhibition des vols en nuées et/ou une inhibition de l'offre, par le mâle, d'une proie à la femelle et/ou une inhibition de la formation par le mâle d'un cocon

Dans la présente par brachycères on entend les insectes appartenant à la famille des muscidés, des oestridés, des hippoboscidés, des nyctéribiides, des syrphides, des tachinides, des thaumatoxénéiidés, des thermitoxénéiidés. Par exemple, il peut s'agir d'insectes choisis dans le groupe comprenant les insectes du genre Musca, Drosophila, Glossina, Calliphora, Auchmeromyia, Lucilia, Sarcophaga, Stomoxys. Il peut s'agir par exemple d'insectes appartenant au genre Drosophila choisi dans le groupe comprenant *Drosophila suzukii, Drosophila melanogaster.*

Dans la présente par « acide propionique », également désigné propanoïque, on entend un acide gras volatile en C3, de formule brute C₃H₆O₂, et/ou de formule semi développée (I) suivante :

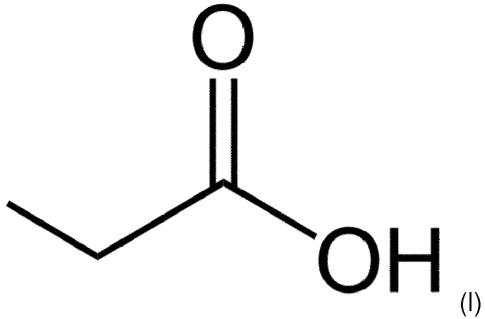

Dans la présente par dérivé de l'acide propionique on entend tout dérivé et/ou sels connus de l'homme du métier. Il peut s'agir par exemple d'un dérivé choisi dans le groupe comprenant un ester d'acide propionique, d'une amine d'acide propionique et/ ou un mélange de ceux-ci.

Selon l'invention, l'ester d'acide propionique peut être tout ester d'acide propionique connu de l'homme du métier. Il peut s'agir par exemple d'un ester de formule CH₃-CH₂-COOR₁ (III) dans lequel R₁ peut être un alkyle en C₁ à C₆, par exemple un méthyle, un éthyle, un butyle, un propyle, un pentyle, un hexyle, un groupement R₁₁ R₁₂ dans lequel R₁₁ est un anion et R₁₂ un cation choisi dans le groupe des métaux alcalins comprenant le lithium, le sodium, le potassium, le rubidium et le césium. Par exemple, l'ester d'acide propionique peut être choisi dans le groupe comprenant le propanoate d'éthyle, le propanoate de propyle, le propanoate de butyle, le propanoate d'isoamyle, le propanoate d'isobutyle et/ou le propanoate d'isopropyle. De préférence l'ester d'acide propionique est le propanoate d'éthyle.

Selon l'invention, l'amine d'acide priopionique peut être toute amine d'acide propionique connue de l'homme du métier. Par exemple l'amine d'acide priopionique peut être choisie dans le groupe comprenant la propylamine, la dipropylamine, la tripropylamine, l'isopropylamine, la diisopropylamine, la triisopropylamine.

Avantageusement, lorsque l'ester est un dérivé de l'acide propionique, de préférence le propanoate d'éthyle ou le butyrate d'éthyle, il présente une fragrance plus agréable pour l'humain que l'acide.

Selon l'invention, l'acide propionique et/ou dérivé de celui-ci peut être à une concentration comprise de 0,1 à 30% en volume (v/v). Selon l'invention, la concentration de l'acide propionique et/ou un dérivé de celui-ci peut être adaptée en fonction du brachycère. Par exemple l'acide propionique et/ou dérivé de celui-ci peut être à une concentration comprise de 0,5% à 1%, de 1% à 2% ou 2% à 30% en volume (v/v). Selon l'invention, la concentration du dérivé d'acide propionique peut être comprise de 2% à 30% en volume (v/v). Par exemple la concentration de propanoate d'éthyle peut être de 10% en volume (v/v).

Dans la présente par « acide butyrique » également dénommé acide butanoïque, on entend un acide gras volatile en C4, de formule brute C₄H₈O₂, et/ou de formule semi-développée (II) suivante :

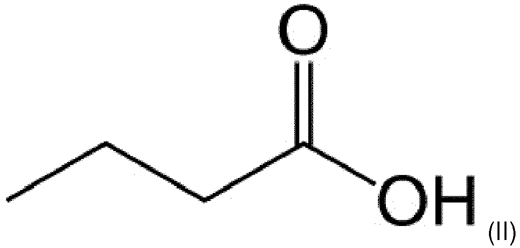

Dans la présente par dérivé de l'acide butyrique on entend tout dérivé et/ou sels connus de l'homme du métier. Il peut s'agir par exemple d'un dérivé choisi dans le groupe comprenant un ester d'acide butyrique et/ou sel de celui-ci.

Selon l'invention, l'ester d'acide butyrique peut être tout ester d'acide butyrique connu de l'homme du métier. Il peut s'agir par exemple d'un ester de formule CH₃-(CH₂)₂-COOR₂ (III) dans lequel R₂ peut être un alkyle en C₁ à C₆, par exemple un méthyle, un éthyle, un butyle, un propyle, un pentyle, un hexyle un groupement R₁₁R₁₂ dans lequel R₁₁ est un anion et R₁₂ un cation choisi dans le groupe des métaux alcalins comprenant le lithium, le sodium, le potassium, le rubidium et le césium. Par exemple l'ester d'acide butyrique peut être choisi dans le groupe comprenant le butyrate d'éthyle, le butyrate de propyle, le butyrate de butyle, le butyrate d'isoamyle, le butyrate d'isobutyle et/ou le butyrate d'isopropyle.

Avantageusement, lorsque qu'il s'agit d'un ester d'acide butyrique, de préférence le butyrate d'éthyle, il présente une fragrance agréable pour l'humain (ananas).

Selon l'invention, l'acide butyrique et/ou dérivé de celui-ci peut être à une concentration comprise de 0,1 à 3% en volume (v/v).

Selon l'invention, la concentration de l'acide butyrique et/ou dérivé de celui-ci peut être adaptée en fonction du brachycère. Par exemple l'acide butyrique et/ou dérivé de celui-ci peut être à une concentration comprise de 0,5% à 3% en volume (v/v). Selon l'invention, la concentration du dérivé d'acide butyrique peut être avantageusement égale à 2% en volume (v/v). Selon l'invention, la concentration de dérivé d'acide butyrique peut être comprise de 2% à 3%. Par exemple lorsque que le dérivé est le butyrate d'éthyle la concentration peut être supérieure ou égale à 2%.

Selon l'invention, lorsque de l'acide propionique et/ou un dérivé de celui-ci et de l'acide butyrique et/ou un dérivé de celui-ci sont utilisés comme principes actifs, la concentration d'acide propionique et/ou du dérivé peut être comprise entre 0,1 et 0,5% en volume (v/v) ; et la concentration d'acide butyrique et/ou du dérivé peut être comprise entre 0,1% et 0,5% en volume (v/v).

La présente invention a également pour objet une composition comprenant au moins un acide gras choisi dans le groupe comprenant l'acide propionique, l'acide butyrique et/ou un dérivé de ceux-ci et un support acceptable.

La présente invention a également pour objet l'utilisation d'une composition comprenant au moins un acide gras choisi dans le groupe comprenant l'acide propionique, l'acide butyrique et/ou un dérivé de ceux-ci et un support acceptable comme répulsif et/ou pour contrôler la reproduction de brachycères.

Selon l'invention, le support peut être tout support connu de l'homme du métier adapté pour être mélangé et/ou associé à des acides gras dans une composition. Il peut s'agir par exemple d'une huile, par exemple une huile minérale, une huile végétale et/ou un mélange de celles-ci. Il peut s'agir par exemple de toute huile minérale connue de l'homme du métier adaptée pour une utilisation dans le domaine pharmaceutique, cosmétique et/ou phytosanitaire. Il peut s'agir par exemple d'une huile minérale choisie dans le groupe comprenant l'huile de paraffine, la vaseline. De préférence, l'huile minérale est l'huile de paraffine. Avantageusement, l'huile de paraffine est sans effet significatif vis-à-vis des brachycères. En outre, l'huile de paraffine permet avantageusement une libération constante du ou des acide(s) gras et/ou dérivé(s) avec laquelle elle est mélangée.

Selon l'invention, l'huile végétale peut être toute huile végétale connue de l'homme du métier adaptée pour une utilisation dans le domaine pharmaceutique, cosmétique et/ou phytosanitaire.

Selon l'invention, la composition peut être une composition cosmétique ou dermatologique, une composition pharmaceutique et/ou une composition phytosanitaire.

Dans la présente par « composition cosmétique » on entend toute forme de composition cosmétique connue de l'homme du métier. La composition cosmétique selon l'invention peut comprendre un ou plusieurs adjuvants et/ou support cosmétiquement acceptable connus de l'homme du métier. Sauf dans le cas où un adjuvants et/ou support cosmétiquement acceptable conventionnel s'avérerait incompatible avec les composés selon l'invention, par exemple en produisant un quelconque effet biologique indésirable et/ou ait une odeur et/ou un effet susceptible de masquer l'odeur des composés selon l'invention, ou bien en interagissant de manière délétère avec tout autre composant de la composition cosmétique, son utilisation est envisagée comme tombant dans le cadre de la présente invention. Il peut s'agir par exemple d'un ou plusieurs adjuvant(s) choisi(s) parmi des agents de type esters, des agents hydratants, des agents émollients, des émulsionnants, des tensio-actifs, des agents épaississants minéraux, des agents épaississants organiques, associatifs ou non, des filtres solaires organiques hydrosolubles et liposolubles, des filtres solaires minéraux, des composés siliconés, des parfums, des conservateurs, des céramides, et pseudo-céramides, des vitamines et des provitamines, des protéines, des agents séquestrants, des agents alcalinisants, des agents acidifiants, des agents réducteurs, des agents oxydants, des charges minérales, des colorants ou tout autre adjuvant adapté pouvant être cité dans le dictionnaire INCl (International Nomenclature of Cosmetic Ingredients) publié par le PCPC (Personal Care Products council)

Selon l'invention, la composition cosmétique ou dermatologique peut, par exemple, se présenter sous toute forme connue de l'homme du métier susceptible d'être utilisée dans le domaine cosmétique. Sauf dans le cas où la formulation de la composition cosmétique ou dermatologique serait incompatible avec les composés selon l'invention, par exemple en produisant un quelconque effet biologique indésirable et/ou une odeur et/ou un effet susceptible de masquer l'odeur des composés selon l'invention et/ou en interagissant de manière délétère avec tout autre composant de la composition cosmétique, son utilisation est envisagée comme tombant dans le cadre de la présente invention. L'homme du métier de part ces connaissances générales saura adapter la formulation en fonction de l'application envisagée.

Dans la présente, la composition cosmétique peut être obtenue par tout procédé approprié connu de l'homme du métier pour la fabrication d'une composition cosmétique. Il peut s'agir d'un mélange de matières tensioactives dans de l'eau.

Dans la présente, par « composition pharmaceutique » on entend toute forme de composition pharmaceutique adaptée connue de l'homme du métier. Dans la présente, la composition pharmaceutique peut être par exemple une solution topique, une forme galénique orodispersible et/ou une solution aérodispersible, par exemple une solution liquide, un spray.

Selon la présente invention, la composition pharmaceutique peut être une composition pharmaceutique pour administration topique, transdermique, par exemple choisie dans le groupe comprenant les pommades, les crèmes, les gels, les lotions, les patchs et les mousses. Sauf dans le cas où la formulation de la composition pharmaceutique serait incompatible avec les composés selon l'invention, par exemple en produisant un quelconque effet biologique indésirable et/ou une odeur et/ou un effet susceptible de masquer l'odeur des composés selon l'invention et/ou en interagissant de manière délétère avec tout autre composant de la composition pharmaceutique, son utilisation est envisagée comme tombant dans le cadre de la présente invention. L'homme du métier de part ces connaissances générales saura adapter la formulation en fonction de l'application envisagée.

Dans la présente, la composition peut être formulée et/ou adaptée selon son administration. La composition de la présente invention peut également comprendre au moins un autre ingrédient actif, particulièrement un autre ingrédient thérapeutiquement actif, par exemple pour une utilisation simultanée, séparée ou étalée dans le temps suivant la formulation galénique utilisée.

Comme décrit ci-dessus, les compositions pharmaceutiquement acceptables de la présente invention peuvent comprendre en outre un support pharmaceutiquement acceptable, un adjuvant ou un véhicule, qui, tel que défini dans la présente, inclut n'importe quel solvant, diluant ou autre véhicule liquide, dispersion ou auxiliaire de suspension, agent tensioactif, agent isotonique, agent épaississant ou émulsifiant, conservateur, liant solide, lubrifiant et analogue, adapté à la forme posologique particulière souhaitée. Remington Pharmaceutical Sciences, seizième édition, E. W. Martin (Mack Publishing Co., Easton, Pa., 1980 [5]) décrit différents supports utilisés dans la formulation de compositions pharmaceutiquement acceptables et des techniques connues pour leur préparation. Sauf dans le cas où un milieu support conventionnel s'avérerait incompatible avec les composés selon l'invention, par exemple en produisant un quelconque effet biologique indésirable et/ou ait une odeur susceptible de masquer l'odeur des composés selon l'invention ou bien en interagissant de manière délétère avec tout autre composant de la composition pharmaceutiquement acceptable, son utilisation est envisagée comme tombant dans le cadre de la présente invention. Quelques exemples de matériaux qui peuvent servir de supports pharmaceutiquement acceptables comprennent, mais ne sont pas limités à, des échangeurs d'ions, des substances tampons, l'eau, des sels ou des électrolytes des cires, des sucres; des amidons, des excipients, des huiles, des glycols, des esters, ainsi que d'autres lubrifiants compatibles non toxiques, ainsi que des agents colorants, des agents de démoulage, des agents d'enrobage, des agents édulcorants, des arômes, des conservateurs et des antioxydants peuvent également être présents dans la composition, selon le jugement du galéniste.

Selon l'invention, lors de son utilisation, l'acide gras choisi dans le groupe comprenant l'acide propionique, l'acide butyrique et/ou un dérivé de ceux-ci et/ou la composition comprenant au moins un acide gras choisi dans le groupe comprenant l'acide propionique, l'acide butyrique et/ou un dérivé de ceux-ci peut être appliqué par tout moyen adapté connu de l'homme du métier. Par exemple, il peut s'agir d'une application au moyen d'un diffuseur, d'un spray, d'un pulvérisateur, d'un support imprégné.

Selon l'invention, le moyen d'application peut être adapté en fonction de la composition utilisée. L'homme du métier, de par ses connaissances générales, saura adapter le moyen d'application à la forme de la composition utilisée.

La présente invention a également pour objet un procédé de protection d'au moins une surface biotique et/ou abiotique vis-à-vis de brachycères comprenant l'application sur ladite surface d'au moins un acide gras choisi dans le groupe comprenant l'acide propionique, l'acide butyrique et/ou un dérivé de ceux-ci ou une composition comprenant au moins un acide gras choisi dans le groupe comprenant l'acide propionique, l'acide butyrique et/ou un dérivé de ceux-ci.

L'acide propionique et/ou un dérivé de celui-ci est tel que défini ci-dessus.

L'acide butyrique et/ou un dérivé de celui-ci est tel que défini ci-dessus.

La composition est telle que définie ci-dessus.

Dans la présente invention par surface on entend toute surface connue de l'homme du métier. Il peut s'agir par exemple d'une surface biotique ou abiotique.

Dans la présente par surface biotique on entend toute surface biotique connue de l'homme du métier. Il peut s'agir par exemple d'une surface biotique choisie dans le groupe comprenant la peau, les muqueuses, les cheveux, les phanères, les yeux, les plumes.

Dans la présente la surface biotique peut être une surface saine et/ou présentant au moins une lésion et/ou une infection. Il peut s'agir par exemple d'une surface biotique comprenant une plaie, une plaie infectée, une plaie en voie de cicatrisation, une plaie suturée, une cicatrice, une brûlure.

Dans la présente la surface biotique peut être une surface de tout ou partie de végétaux et/ou de plante. Il peut s'agir par exemple de la surface de feuilles, de fruits, de fleurs, de pétales, de pistils, de tiges, de branches, de troncs.

Dans la présente par surface abiotique on entend toute surface abiotique connue de l'homme du métier. Il peut s'agir également de toute surface de dispositif connue de l'homme du métier. Il peut s'agir par exemple de la surface d'un dispositif médical, de toute surface présente dans les salles/pièces d'urgences médicales, de traitements médicaux, d'opération, les blocs opératoires, les salles/pièces des unités de soins intensifs (UTI), les salles/pièces des unités de maladies infectieuses. Il peut s'agir également de toute surface des salles/pièces de laboratoire, de laboratoire biologique, de laboratoire d'analyses biologiques, de salle d'incubation et d'un autre volume clos. Il peut s'agir par exemple de la surface de bâtiment, par exemple d'habitation, de stockage et/ou agricole. Il peut s'agir par exemple de la surface de toitures, de murs, de fenêtres, de gouttières.

Dans la présente, la surface abiotique peut être formée par tout matériau connu de l'homme du métier. Il peut s'agir par exemple d'un matériau biocompatible ou non. Dans la présente, la surface abiotique peut être par exemple une surface métallique, une surface formée par un alliage, une surface polymérique. Par exemple, le métal peut être tout métal connu de l'homme du métier, par exemple un métal choisi dans le groupe comprenant le titane, le cuivre, le fer, l'aluminium, le nickel, le tungstène, l'argent, l'or, le palladium, le vanadium, le molybdène. Par exemple, l'alliage peut être tout alliage connu de l'homme du métier, par exemple un alliage choisi dans le groupe comprenant l'acier, le laiton, le cuivre-nickel, le cuivre-palladium, l'argent-or, l'argent-palladium, le molybdène-vanadium, le molybdène-tungstène. Par exemple, le polymère peut être tout polymère connu de l'homme du métier susceptible de constituer et/ou de recouvrir une surface. Il peut s'agir par exemple d'un polymère choisi dans le groupe comprenant le polytétrafluoroéthylène (PTFE), les polysiloxanes, les polyuréthanes, les polymères fonctionnalisés.

Selon l'invention, l'application peut être réalisée par tout moyen connu de l'homme du métier. Par exemple l'application peut être réalisée avec un diffuseur, un pulvérisateur, un diffuseur sous pression. L'homme du métier de par ses connaissances générales saura adapter et/ou choisir le moyen d'application en fonction de la surface.

Avantageusement, lorsque les acides gras selon l'invention et/ou la composition selon l'invention sont/est appliqués(ée) sur une surface abiotique, ils permettent d'éviter l'apparition de brachycères à proximité de ces surfaces, en particulier, ils permettent de repousser et/ou d'éloigner les brachycères desdites surfaces.

La présente invention a également pour objet un kit ou une trousse contenant au moins un acide gras choisi parmi l'acide propionique et/ou un dérivé de celui-ci, l'acide butyrique et/ou un dérivé de celui-ci, et/ou une composition comprenant au moins un acide gras choisi parmi l'acide propionique et/ou un dérivé de celui-ci, l'acide butyrique et/ou un dérivé de celui-ci, et un support comprenant des instructions d'utilisations.

L'acide propionique et/ou un dérivé de celui-ci est tel que défini ci-dessus.

L'acide butyrique et/ou un dérivé de celui-ci est tel que défini ci-dessus.

La composition est telle que définie ci-dessus.

D'autres avantages pourront encore apparaître à l'homme du métier à la lecture des exemples ci-dessous, illustrés par les figures annexées, données à titre illustratif.

### Brève description des figures

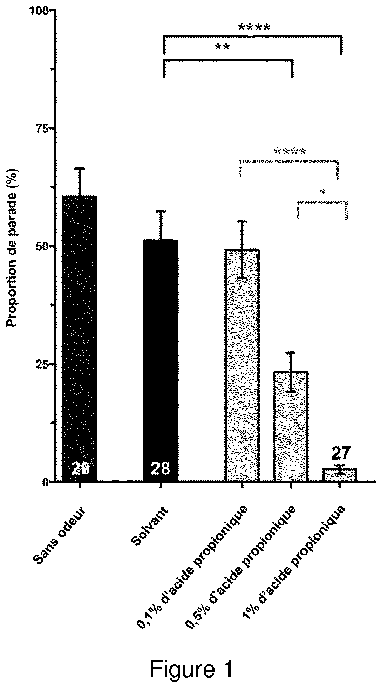

- La figure 1 est un diagramme en bâtons représentant la mesure de la proportion en pourcentage de parade de *Drosophila suzukii* mâles à l'égard de femelles (ordonnées), en l'absence ou en présence d'une composition comprenant 0,1%, 0,5% et 1% d'acide propionique (abscisses). Sur la figure (*), (**), (***) et (****) signifient une différence statistique entre les différentes conditions présentant des quantités variables d'acide propionique et calculée en effectuant un test de Kruskall-Wallis, suivi d'un test post-hoc de Dunn à comparaisons multiples.

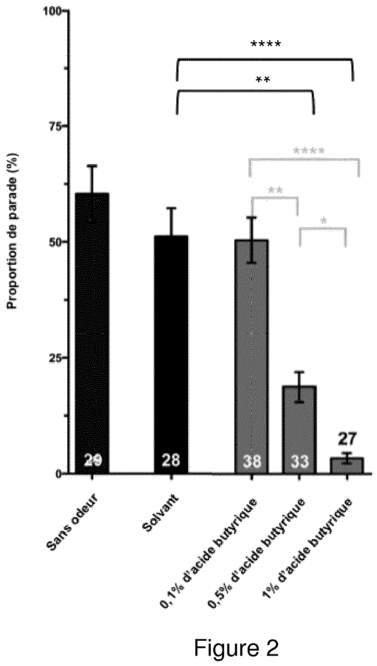

- La figure 2 est un diagramme en bâtons représentant la mesure de la proportion en pourcentage de parade de *Drosophila suzukii* mâles à l'égard de femelles (ordonnées) en l'absence ou en présence d'une composition comprenant 0,1%, 0,5% et 1% d'acide butyrique (abscisses). Sur la figure (*), (**), (***) et (****) signifient une différence statistique entre les différentes conditions présentant des quantités variables d'acide butyrique et calculée en effectuant un test de Kruskall-Wallis, suivi d'un test post-hoc de Dunn à comparaisons multiples.

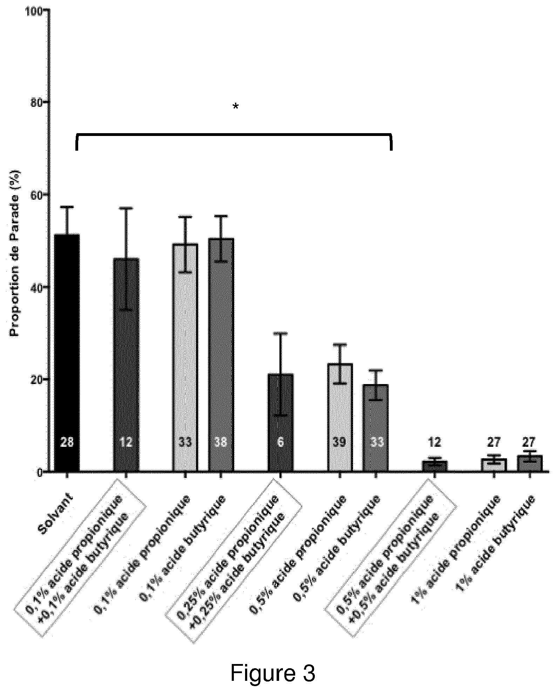

- La figure *3* *est un diagramme* en bâtons représentant la mesure de la proportion en pourcentage de parade de *Drosophila suzukii* mâles à l'égard de femelles (ordonnées) en l'absence ou en présence d'une composition comprenant respectivement 0,1%, 0,5% et 1% d'acide propionique ou 0,1%, 0,5% et 1% d'acide butyrique ou des mélanges correspondant à 0,1%, 0,25% ou 0,5% d'acide propionique et d'acide butyrique (abscisses). Sur la figure (*) signifie une différence statistique entre les différentes conditions présentant des quantités variables d'acides gras. Le test statistique effectué est un test de Kruskall-Wallis, suivi d'un test post-hoc de Dunn à comparaisons multiples.

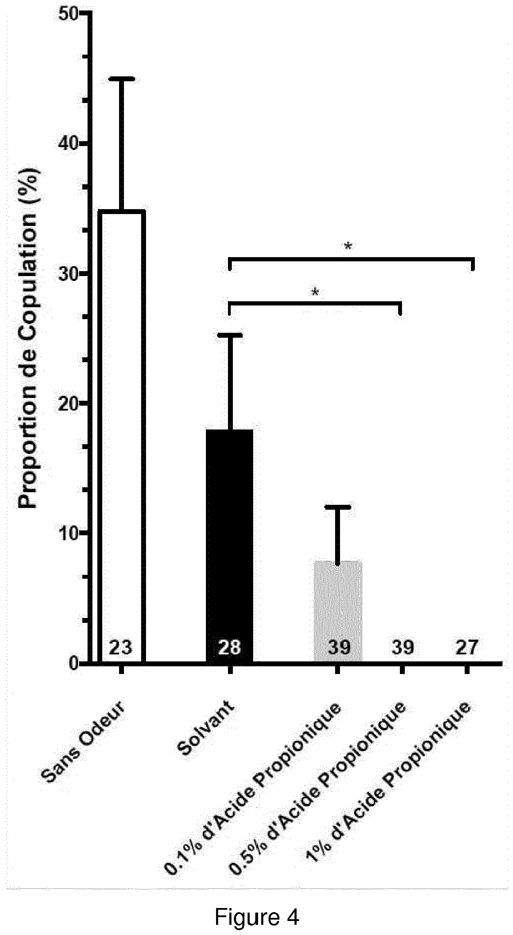

- La figure 4 est un diagramme en bâtons représentant la mesure de la proportion en pourcentage de copulation de *Drosophila suzukii* (ordonnées) en l'absence ou en présence d'une composition comprenant respectivement 0,1%, 0,5% et 1% d'acide propionique (abscisses). Sur la figure (*) signifie une différence statistique entre les différentes conditions présentant des quantités variables d'acide propionique. Le test statistique effectué est un test de Kruskall-Wallis, suivi d'un test post-hoc de Dunn à comparaisons multiples.

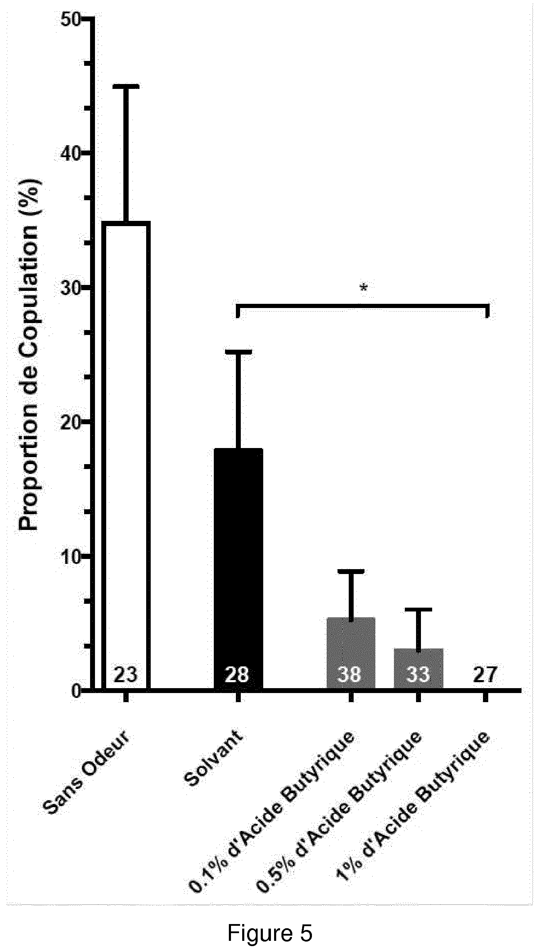

- La figure 5 est un diagramme en bâtons représentant la mesure de la proportion en pourcentage de copulation de *Drosophila suzukii* (ordonnées) en l'absence ou en présence d'une composition comprenant respectivement 0,1%, 0,5% et 1% d'acide butyrique (abscisses). Sur la figure (*) signifie une différence statistique entre les différentes conditions présentant des quantités variables d'acide butyrique. Le test statistique effectué est un test de Kruskall-Wallis, suivi d'un test post-hoc de Dunn à comparaisons multiples.

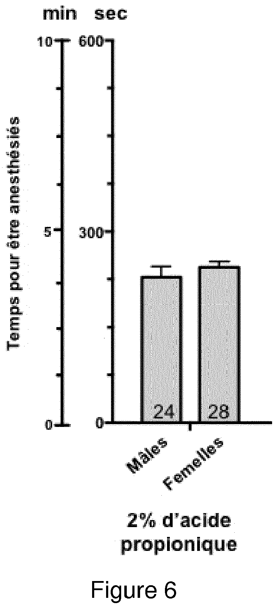

- La figure 6 est un diagramme en bâtons représentant le temps écoulé conduisant à l'anesthésie de *Drosophila suzukii* mâles ou femelles (abscisses) en minutes et en secondes (ordonnées) en présence d'une composition comprenant 2% d'acide propionique. Un test de Mann-Whitney (test t non paramétrique) ne révèle pas de différence liée au temps conduisant à l'anesthésie des mouches selon leur sexe.

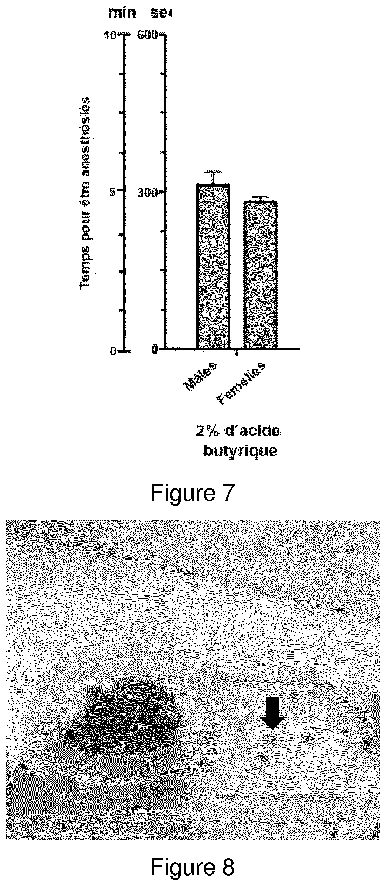

- La figure 7 est un diagramme en bâtons représentant le temps écoulé conduisant à l'anesthésie de *Drosophila suzukii* mâles ou femelles (abscisses) en minutes et en secondes (ordonnées) en présence d'une composition comprenant 2% d'acide butyrique. Un test de Mann-Whitney (test t non paramétrique) ne révèle pas de différence liée au temps conduisant à l'anesthésie des mouches selon leur sexe.
- La figure 8 est une photographie représentant une partie de l'intérieur d'une boîte de test transparente de 1250 cm³ dans laquelle de l'acide propionique à 2% a été déposé dans une boîte de Pétri recouverte d'une gaze (en dehors du cadre de l'image représentée). Les points noirs sur la figure correspondent aux mouches *Drosophila suzukii.* La flèche indique une mouche *Drosophila suzukii* sur le dos, en position d'anesthésie.

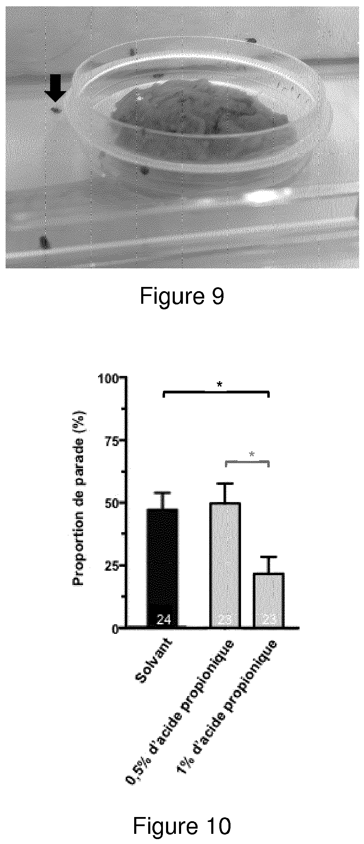

- La figure 9 est une photographie représentant une partie de l'intérieur d'une boîte de test transparente de 1250 cm³ dans laquelle de l'acide butyrique à 2% a été déposé dans une boîte de Pétri recouverte d'une gaze (en dehors du cadre de l'image représentée). Les points noirs sur la figure correspondent aux mouches *Drosophila suzukii.* La flèche indique une mouche *Drosophila suzukii* sur le dos, en position d'anesthésie.
- La figure 10 est un diagramme en bâtons représentant la mesure de la proportion en pourcentage de parade de *Drosophila melanogaster* (ordonnées) en l'absence ou en présence d'une composition comprenant respectivement 0,5% et 1% d'acide propionique (abscisses). Sur la figure (*) signifie une différence statistique entre les différentes conditions présentant des quantités variables d'acide propionique et calculée en effectuant un test de Mann-Whitney (test non paramétrique).

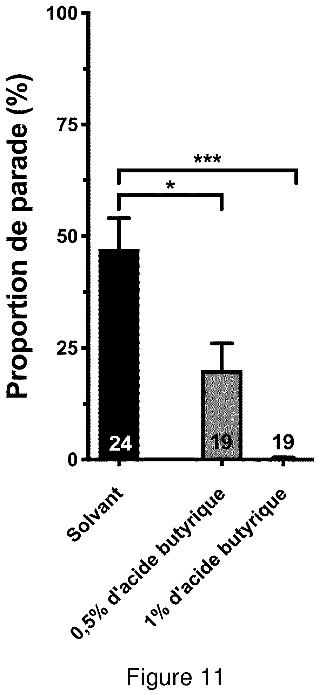

- La figure 11 est un diagramme en bâtons représentant la mesure de la proportion en pourcentage de parade de *Drosophila melanogaster* (ordonnées) en l'absence ou en présence d'une composition comprenant respectivement 0,5% et 1% d'acide butyrique (abscisses). Sur la figure, (***) signifie une différence statistique entre les différentes conditions présentant des quantités variables d'acide butyrique et calculée en effectuant un test de Mann-Whitney (test non paramétrique).

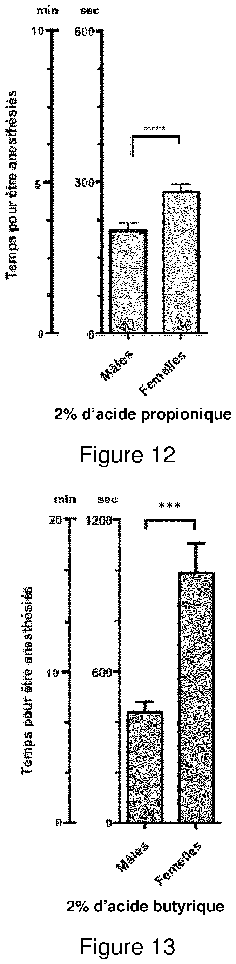

- La figure 12 est un diagramme en bâtons représentant le temps écoulé conduisant à l'anesthésie de *Drosophila melanogaster* mâles ou femelles (abscisses) en minutes et en secondes (ordonnées) en présence d'une composition comprenant 2% d'acide propionique. Sur la figure (****) signifie une différence statistique entre les différentes conditions présentant des quantités variables d'acide propionique et calculée en effectuant un test de Mann-Whitney (test non paramétrique).
- La figure 13 est un diagramme en bâtons représentant le temps écoulé conduisant à l'anesthésie de *Drosophila melanogaster* mâles ou femelles (abscisses) en minutes et en secondes (ordonnées) en présence d'une composition comprenant 2% d'acide butyrique. Sur la figure (***) signifie une différence statistique entre les différentes conditions présentant des quantités variables d'acide propionique et calculée en effectuant un test de Mann-Whitney (test non paramétrique).

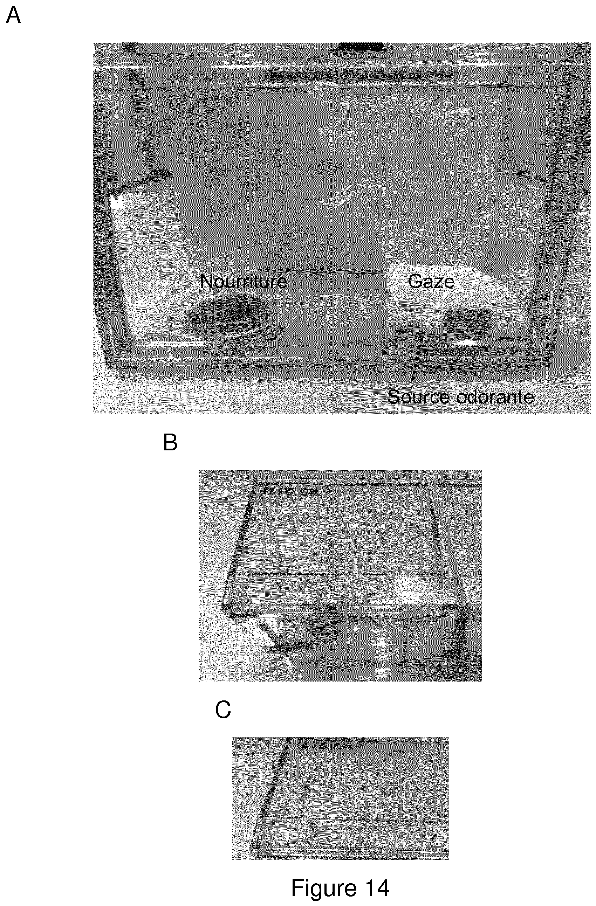

- La figure 14 correspond à trois photographies d'une même boîte de test (dans son ensemble figure 14A et en partie vue du dessus (figure 14B et figure 14C)) utilisée pour l'étude du comportement de répulsion et/ou d'anesthésie à distance de *D. melanogaster* et *D*. *suzukii.* Dans cette boîte transparente d'un volume de 1250 cm³, 2 boîtes de Pétri sont déposées : l'une, recouverte d'une gaze (visible à droite de l'image montrant la boîte de test dans son ensemble), contenait de l'acide propionique à 2%, 6% ou 20% et l'autre contenait de la nourriture synthétique (visible à gauche de l'image). Les points noirs correspondent aux mouches *Drosophila melanogaster.*

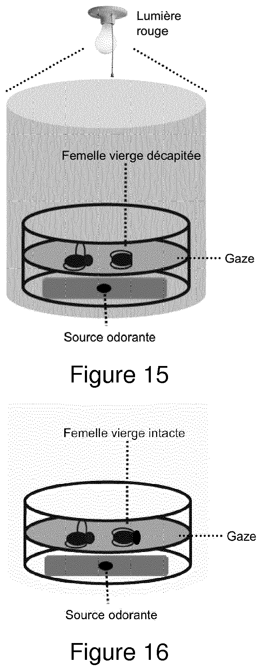

- La figure 15 est un schéma représentant une des six alvéoles cylindriques du dispositif de comportement utilisé pour l'étude de la parade de *D. melanogaster.* A la base du cylindre est déposé un papier filtre imbibé d'une odeur, la partie centrale maintient un voilage sur laquelle se déplacent les mouches testées, ce cylindre est finalement clos. Pour les tests de parade de *D. melanogaster,* une lumière rouge éclaire le dispositif.
- La figure 16 est un schéma représentant un dispositif de comportement utilisé pour l'étude de la parade de *D. suzukii.* Il est similaire à celui utilisé pour *D*. *melanogaster* mais est éclairé par de la lumière blanche.

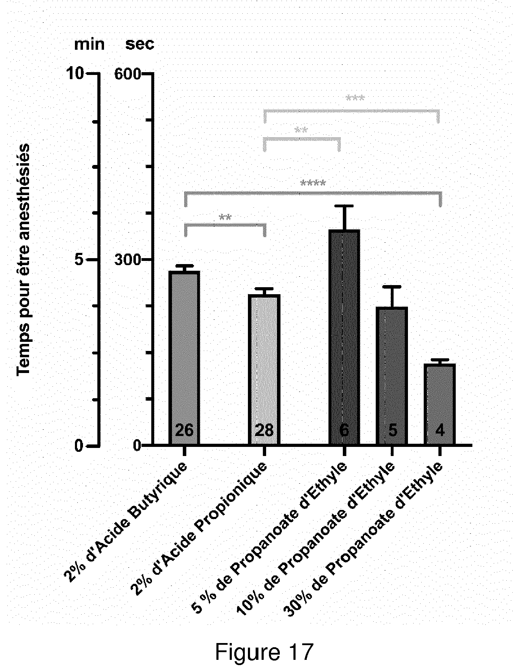

- La figure 17 est un diagramme en bâtons représentant le temps écoulé conduisant à l'anesthésie de *Drosophila suzukii* femelles en minutes et en secondes (ordonnées) en présence d'une composition comprenant 2% d'acide butyrique, 2% d'acide propionique, 5% de propanoate d'éthyle, 10% de propanoate d'éthyle ou 30% de propanoate d'éthyle (abscisses).

### EXEMPLES

### Exemple 1 : Effet d'acide propionique et/ou d'acide butyrique sur la reproduction de brachycères

Dans cet exemple, les brachycères utilisées étaient les mouches (muscinés) *Drosophila melanogaster* et *Drosophila suzukii.* Dans cet exemple, une étude de la parade et de la copulation a été réalisée.

### A) Matériel et méthode

### 1. Evaluation de la parade

### 1.1. Drosophila melanogaster

Des drosophiles (*Drosophila melanogaster*) âgées de 3 à 9 jours sont utilisées pour les tests de parade tels que décrits dans Grosjean et al. Nat. Neurosci. 2008 [1] et Grosjean et al. Nature 2011 [2].

Des mâles naïfs ont été isolés dans des tubes, individuellement, à l'émergence de leur puparium. Les femelles ont été choisies vierges et stockées en tubes de 10 individus. Ces tubes ont été placés dans des incubateurs pour lesquels une température de 25°C est maintenue constante. Un cycle jour-nuit, 12h - 12h a été appliqué. Les tests de parade ont été effectués le matin, entre 1 et 3 heures après le début du cycle jour.

Ces tests ont été effectués dans des dispositifs constitués de 6 alvéoles cylindriques, transparentes et closes (voir figure 15) d'un volume de 1,52 cm³. A leur base un disque de papier filtre (Whatman, filter paper 42) de 6 mm de diamètre est déposé. Ledit papier est imbibé de 10 µl d'une composition comprenant de l'acide propionique et/ou de l'acide butyrique diluée dans de l'huile de paraffine (solvant). Les différentes compositions utilisées comprenaient respectivement 0,5% ou 1% d'acide propionique, 0,5% ou 1% d'acide butyrique. Pour chaque concentration, le nombre de couples de mouches testé étaient respectivement de 23, 23, 19 et 19.

Au moment du test, 1 couple par alvéole a été introduit dans le dispositif de comportement (soient 6 couples). Ce couple était composé d'un mâle et d'une femelle, chacun issu des tubes mentionnés précédemment. Les drosophiles ont été isolées physiquement de la source d'odeur (ledit papier) par un voilage qui empêche tout contact direct, la femelle a été décapitée mais est restée vivante. Aussi, le dispositif a été éclairé par une lumière rouge (rouge lointain de longueur d'onde comprise de 625 et 630 nm), de façon à éliminer tout comportement lié à la perception visuelle, comprise chez *D. melanogaster* entre 300 et 550 nm)). Ainsi, le comportement observé dépend uniquement de la perception olfactive par le mâle du composé introduit dans le dispositif au moment de son assemblage.

Le comportement a été filmé avec une caméra Sony HDR-XR550 pendant 10 minutes. Chaque film a été enregistré et conservé pour son analyse ultérieure.

Une proportion de parade exprimée en pourcentage a ensuite été calculée et représente le temps durant lequel le mâle parade la femelle au cours des 10 minutes d'enregistrement du test. La parade des *Drosophila melanogaster* est décomposée en événements stéréotypés reconnaissables et décrits précisément dans la littérature scientifique (Greenspan & Ferveur, Annu. Rev. Genet. 2000 |3]). Un test statistique (test de Mann-Whitney (test non paramétrique, GraphPad, Prism8)) afin d'évaluer l'influence sur le comportement de la composition imbibée sur le papier a été effectué ; cette dernière impliquant une libération de composés volatiles et une odeur perçue par les drosophiles.

Un contrôle négatif a été réalisé et correspondait en l'imbibition du papier filtre avec une solution d'huile de paraffine seule (le solvant) afin de vérifier l'absence d'effet sur les mouches.

### 1.2. Drosophila suzukii

Des drosophiles (*Drosophila suzukii)* âgées de 3 à 9 jours sont utilisées pour les tests de parade selon le procédé décrit ci-dessus.

Des mâles naïfs ont été isolés dans des tubes, individuellement, à l'émergence de leur puparium. Les femelles ont été choisies vierges et stockées en tubes de 10 individus. Ces tubes ont été placés inclinés dans des incubateurs pour lesquels une température de 25°C a été maintenue constante. Un cycle jour-nuit, 12h - 12h a été appliqué. Les tests de parade ont été effectués le matin, entre 1 et 3 heures après le début du cycle jour.

Au moment du test, un disque en papier filtre (Whatman, filter paper 42) de 6 mm a été déposé par alvéole du dispositif d'étude du comportement de parade. Chaque alvéole était cylindrique, transparente et close, et d'un volume de 1,52 cm³. La figure 16 est une représentation schématique d'une alvéole du dispositif utilisé.

Le papier a été ensuite imbibé de 10µl d'une composition de 0,1%, 0,5% ou 1% d'acide propionique, ou de 0,1%, 0,5% ou 1% d'acide butyrique ou d'un mélange comprenant 0,1% d'acide propionique et 0,1% d'acide butyrique, 0,25% d'acide propionique et 0,25% d'acide butyrique ou 0,5% d'acide propionique et 0,5% d'acide butyrique. Une gaze (voilage) permet d'isoler physiquement les drosophiles de l'odeur. 6 couples (un par alvéole) ont été observés indépendamment. Chaque couple était composé d'un mâle et d'une femelle intacte, chacun issu des tubes mentionnés précédemment.

Le comportement a été filmé avec une caméra Sony HDR-XR550 pendant 10 minutes, sous lumière blanche. Chaque film a été enregistré puis analysé. Pour chaque concentration des compositions utilisées, le nombre de couple de mouches étaient respectivement de 33, 39, 27, 38, 33, 27, 12, 6 et 12.

Un contrôle négatif a été réalisé comprenant uniquement le solvant, à savoir de l'huile de paraffine, afin de vérifier l'absence d'effet du solvant sur les mouches.

Un pourcentage de parade a été calculé et représente le temps durant lequel le mâle parade la femelle au cours des 10 minutes d'observation (enregistrement). La parade des *Drosophila suzukii* est décomposée en événements stéréotypés reconnaissables et décrits précisément dans la littérature scientifique (Revadi S et al., Insects 2015 [4]). Des tests statistiques, à savoir un test de Kruskal-Wallis suivi d'un test post-hoc de Dunn à comparaisons multiples, ont été effectués (GraphPad, Prism6) afin d'évaluer l'influence sur le comportement de la composition imbibée sur le papier ; cette dernière impliquant une libération de composés volatiles et une odeur perçue par les drosophiles.

### 2. Evaluation de la Copulation

A partir des films enregistrés pour l'étude du comportement de parade de *Drosophila suzukii,* un pourcentage du nombre de couples copulant pendant les 10 minutes d'observation a été calculé en fonction de la composition imbibée sur le papier filtre. Une évaluation statistique a été réalisée et différents tests ont été effectués. En particulier, un test de Kruskall-Wallis, suivi d'un test post-hoc de Dunn à comparaisons multiples ont été effectués (GraphPad, Prism6) afin de quantifier l'influence de la composition imbibée sur le papier impliquant une libération de composés volatiles et une odeur perçue sur le nombre d'individus copulant.

### B) Résultats

### 1. Evaluation de la parade

Les figures 1 à 3 représentent les résultats obtenus avec des mouches *Drosophila suzukii* en fonction respectivement notamment de la présence ou de l'absence d'acide propionique, d'acide butyrique ou d'un mélange d'acide propionique et d'acide butyrique.

Tel que représenté sur la Figure 1, en présence de 0,5% d'acide propionique dans le solvant, la parade était significativement diminuée par rapport au solvant seul (**). En présence de 1% d'acide propionique dans le solvant, la parade était significativement diminuée par rapport au solvant seul (****) ; de même comparée à une composition comprenant 1% (****) *vs. 0* ,1 % ou 1% *vs.* 0,5% d'acide propionique (*).

Tel que représenté sur la Figure 2, en présence de 0,5% d'acide butyrique dans le solvant, la parade est significativement diminuée par rapport au solvant seul (**) ; de même, comparée à 0,1% d'acide butyrique dans le solvant (**).

En présence de 1% d'acide butyrique dans le solvant, la parade est significativement diminuée par rapport au solvant seul (****) ; de même comparée à 0,1% (****) et à 0,5% (*).

Tel que représenté sur la figure 3, en présence de 0,25% d'acide propionique et de 0,25% d'acide butyrique dans le solvant, la parade était diminuée par rapport au solvant seul. En présence de 0,5% d'acide propionique et de 0,5% d'acide butyrique dans le solvant, la parade était significativement diminuée comparée à 0,1% d'acide propionique et à 0,1% d'acide butyrique dans le solvant (*) et à 0,5% d'acide propionique et de 0,5% d'acide butyrique dans le solvant.

Les figures 10 et 11 représentent les résultats obtenus avec des mouches *Drosophila melanogaster* en fonction respectivement notamment de la présence ou de l'absence d'acide propionique ou d'acide butyrique.

Tel que représenté sur la figure 10, la proportion de parade des mâles à l'égard de femelles était significativement modifiée (*) en présence de la composition comprenant 1% d'acide propionique par rapport au solvant seul. La parade était également significativement diminuée (*) en présence de 1% d'acide propionique dans le solvant par rapport à 0,5% d'acide propionique dans le solvant.

Tel que représenté sur la figure 11, la proportion de parade des mâles à l'égard de femelles était significativement diminuée (*) en présence d'une composition comprenant 0,5% d'acide butyrique comparée à la parade dans le solvant seul. La parade était également significativement diminuée (***) en présence d'une composition comprenant 1% d'acide butyrique comparée à la parade dans le solvant seul.

### 2. Evaluation de la copulation

Les figures 4 et 5 représentent les résultats obtenus avec des mouches *Drosophila suzukii* en fonction respectivement de la présence ou de l'absence d'acide propionique, d'acide butyrique ou d'un mélange d'acide propionique et d'acide butyrique.

Tel que représenté sur la Figure 4 la proportion de copulation était diminuée de 58% en présence d'une composition comprenant 0,1% d'acide propionique dilué dans le solvant. En présence d'une composition comprenant 0,5% d'acide propionique ((*) *vs.* solvant seul) ou 1% d'acide propionique ((*) *vs.* solvant seul), la proportion de copulation est nulle, aucune copulation n'a été observée.

Tel que représenté sur la Figure 5, la proportion de copulation était diminuée de 70,55% en présence d'une composition comprenant 0,1% d'acide butyrique. En présence d'une composition comprenant 0,5% d'acide butyrique, la copulation était diminuée de 83,03% (passant de 17,86% à 3,03%), et en présence d'une composition comprenant 1% d'acide butyrique ((*) *vs.* solvant seul), la proportion de copulation est nulle, aucune copulation n'a été observée.

Tel que démontré ci-dessus, la présence d'acide butyrique et/ou d'acide propionique permet de contrôler la reproduction de brachycères. En particulier, tel que démontré ci-dessus, des exemples de compositions selon l'invention permettent à la fois de réduire la parade des mâles vis-à-vis des femelles et également de réduire voire de supprimer la copulation de brachycères.

Cet exemple démontre également que l'acide propionique, l'acide butyrique et/ou un dérivé de ceux-ci sont utiles comme principe actif pour contrôler la reproduction de brachycères.

Cet exemple démontre en outre que l'acide propionique, l'acide butyrique et/ou un dérivé de ceux-ci sont utiles comme principe actif pour contrôler la reproduction de brachycères à des concentrations faibles permettant avantageusement l'obtention d'un effet rapide tout en réduisant et/ou évitant tout éventuel effet secondaire lié, par exemple, à des concentrations importantes.

### Exemple 2 : Effet d'acide propionique et/ou d'acide butyrique et/ou d'un dérivé de l'acide propionique sur la reproduction de brachycères et/ou comme principes actifs répulsifs

Dans cet exemple, les brachycères utilisées étaient les mouches *Drosophila melanogaster* et *Drosophila suzukii.* Les drosophiles (*Drosophila melanogaster* et *Drosophila suzukii*) étaient telles que décrites dans l'exemple 1, les femelles *D. melanogaster* n'étaient en revanche pas décapitées.

Dans cet exemple une étude d'un effet anesthésiant et d'un effet répulsif de l'acide propionique et/ou de l'acide butyrique a été réalisée.

### A) Matériel et méthode

### 1. Tests d'anesthésie :

Les dispositifs utilisés étaient similaires aux dispositifs d'étude de la parade (exemple 1 ci-dessus), excepté le volume de l'espace dans lequel les mouches ont été introduites qui était de 45,24 cm³. 9 disques de papier filtre (Whatman, filter paper 42) imbibé chacun de 10 µl d'une composition comprenant de l'acide butyrique et/ou de l'acide propionique et/ou d'un dérivé de l'acide propionique diluée dans de l'huile de paraffine ont été déposés dans le dispositif. Les différentes compositions utilisées comprenaient respectivement 2% d'acide propionique ou 2% d'acide butyrique ou 5 % de propanoate d'éthyle ou 10% de propanoate d'éthyle ou 30 % de propanoate d'éthyle. Pour chaque composition, le nombre de mouches étaient respectivement pour *Drosophila melanogaster* de 30 et 24 mâles, 30 et 11 femelles ; et pour *Drosophila suzukii* de 24, 16 et 4 mâles, 28, 26, 6, 5 et 4 femelles. Afin d'éviter tout contact des mouches avec les disques en papier imbibés, une gaze (voilage) a été disposée au-dessus desdits papiers. Les drosophiles pouvaient se déplacer librement sur cette gaze. La quantité de composé, d'acide butyrique ou d'acide propionique ou de propanoate d'éthyle (odeur) volatilisée dans la chambre de comportement étaient équivalente à celle du dispositif utilisé pour la parade. De même, les mouches n'étaient jamais en contact direct avec la composition (pas de perception gustative), uniquement avec les particules volatiles (perception olfactive). Les mouches testées ont été introduites simultanément par groupe dans l'enceinte. Leur comportement a été filmé pendant 10 minutes et un temps requis pour conduire à l'anesthésie a été calculé sur cette période, en fonction de la concentration d'acide butyrique ou d'acide propionique ou de propanoate d'éthyle utilisée. Les mouches étaient considérées comme anesthésiées lorsqu'elles cessaient de bouger et tombaient sur le côté. Un test de Mann-Whitney (test non paramétrique, GraphPad, Prism 6 ou Prism 8) n'a pas révélé de différence de temps écoulé avant l'anesthésie des mâles ou des femelles testés.

### 2. Tests "en boîte" (effet répulsif)

La sensibilité des drosophiles a été évaluée sur une durée comprise de 0 min à 24 heures selon les conditions (voir résultats) dans des boîtes closes et transparentes de 1250 cm³.

Une source d'odeur, à savoir une composition comprenant de l'acide butyrique ou de l'acide propionique dans de l'huile de paraffine, a été déposée sur un papier filtre au fond d'une boîte de Pétri de 50 mm. La boîte de Pétri est introduite dans la boîte de tests et recouverte d'une gaze (compresse de gaze stérile Securimed M8202) afin d'éviter aux mouches de s'engluer au composé odorant huileux.

Les concentrations utilisées étaient 2%, 6% ou 20% d'acide propionique.

Une seconde boîte de Pétri contenant 5 à 6 grammes de nourriture synthétique utilisée pour l'élevage des drosophiles en laboratoire, a été mise dans la boîte de tests, à savoir 6,5% de farine de maïs, 6,5% (v/v) d'extrait de levure, 1% (v/v) d'agar-agar et 3% (v/v) d'une solution à 0,1% (v/v) d'antifongique (Tegosept (Apex)) dans de l'éthanol à 95%. Cette dernière apporte l'eau et/ou la nourriture dont les mouches avaient besoin au cours du test et éliminait une possibilité d'un événement dû à une déshydratation des individus testés.

Des *Drosophila melanogaster* ou *suzukii* ont été ensuite introduites par groupes dans la boîte de tests et s'y déplaçaient librement au cours du temps. Leur comportement en présence d'une composition telle que mentionnée ci-dessus a été observé et photographié (Apple iPhone 6S).

### B) Résultats

### 1. Tests d'anesthésie :

Les figures 6 à 9 et la figure 17 représentent les résultats obtenus avec des mouches *Drosophila suzukii* en fonction respectivement de la présence ou de l'absence d'acide propionique ou d'acide butyrique ou de propanoate d'éthyle.

Tel que représenté sur la figure 6, en présence de 2% d'acide propionique, les mouches mâles et les mouches femelles ont été anesthésiées en environ 4 minutes. Une évaluation statistique (Test de Mann-Whitney, p=0,7056) a démontré aucune différence significative du temps nécessaire pour que mâles ou femelles soient anesthésiés.

Tel que représenté sur la figure 7, en présence de 2% d'acide butyrique, les mouches mâles et les mouches femelles ont été anesthésiées en environ 5 minutes. Une évaluation statistique (Test de Mann-Whitney, p=0,43) a démontré aucune différence significative du temps nécessaire pour que mâles ou femelles soient anesthésiés.

La figure 8 est une photographie démontrant l'anesthésie de mouches *Drosophila suzukii* après leur introduction dans une boîte de test transparente de 1250 cm³, où de l'acide propionique dilué à 2% dans le solvant a été déposé dans une boite de Pétri recouverte d'une gaze afin d'éviter tout contact direct. Les individus restaient dans un premier temps, préférentiellement à l'écart de cette odeur, puis les mâles et les femelles ont été anesthésiés dans l'heure qui a suivi. La photographie indique l'emplacement des mouches et un individu sur le dos, en position d'anesthésie dans l'heure suivant son introduction dans la boîte.

La figure 9 est une photographie démontrant l'anesthésie de mouches *Drosophila suzukii* après leur introduction dans une boîte de test transparente de 1250 cm³, où de l'acide butyrique dilué à 2% dans le solvant a été déposé dans une boite de Pétri recouverte d'une gaze afin d'éviter tout contact direct. Les mouches présentent dans la boîte de test transparente sont restées à l'écart du filtre imbibé d'acide butyrique, les mâles et femelles sont anesthésiées et meurent après 20 heures dans cet environnement. La photographie indique l'emplacement des mouches et un individu sur le dos, en position d'anesthésie dans l'heure suivant son introduction dans la boîte

Les figures 12 et 13, obtenues dans les dispositifs décrits en A1, représentent les résultats obtenus avec des mouches *Drosophila melanogaster* en fonction respectivement notamment de la présence ou de l'absence d'acide propionique ou d'acide butyrique.

Tel que représenté sur la figure 12, en présence de 2% d'acide propionique, les mouches mâles ont été anesthésiées en environ 3 minutes et les mouches femelles ont été anesthésiées en environ 5 minutes. Une évaluation statistique (test de Mann-Whitney non paramétrique) entre le temps nécessaire pour que les mouches mâles ou femelles soient anesthésiés a montré une sensibilité supérieure des mâles (****).

Tel que représenté sur la figure 13, en présence de 2% d'acide butyrique, les mouches mâles ont été anesthésiées en environ 5 minutes et les mouches femelles ont été anesthésiés en environ 17 minutes. Une évaluation statistique (Mann-Whitney non paramétrique) entre le temps nécessaire pour que les mouches mâles ou femelles soient anesthésiés a montré une sensibilité supérieure des mâles (****).

La figure 17 obtenue dans les dispositifs décrits en A1, représentent les résultats obtenus avec des mouches femelles *Drosophila suzukii* en présence de 2% d'acide butyrique ou 2% d'acide propionique ou 5 % de propanoate d'éthyle ou 10% de propanoate d'éthyle ou 30 % de propanoate d'éthyle.

Tel que représenté sur la Figure 17, en présence de 2% d'acide butyrique, les mouches femelles ont été anesthésiées en environ 5 minutes et en présence de 2% d'acide propionique les mouches femelles ont été anesthésiées en environ 4 minutes. Une évaluation statistique (Mann-Whitney non paramétrique) du temps nécessaire aux femelles pour être anesthésiées montre une sensibilité supérieure (**) en présence de 2% d'acide butyrique dans le solvant comparée à 2% d'acide propionique dans le solvant. En présence de 5 % de propanoate d'éthyle les mouches femelles ont été anesthésiées en environ 6 minutes, en présence de 10 % de propanoate d'éthyle les mouches femelles ont été anesthésiées en environ 4 minutes ne révélant pas de différence statistique par rapport à 2% d'acide propionique et à 2% d'acide butyrique. En outre, en présence de 30 % de propanoate d'éthyle les mouches femelles ont été anesthésiées en environ 2-3 minutes ce qui correspond à une différence statistique significative par rapport à 2% d'acide butyrique (****) et par rapport à 2% d'acide propionique (***).

### 2 Tests de l'effet répulsif (tests "en boîte")

La figure 14 représente les résultats obtenus avec des mouches *Drosophila Melanogaster.* Dans une boîte où de l'acide propionique dilué à 2% ou à 6% dans le solvant (huile de paraffine), les mouches sont toujours vivantes après 24 heures. Au cours de cette période, mâles et femelles paradent et copulent (figure 14) ; ôtés de la boîte ils auront une descendance viable.

A 20% d'acide propionique dilué dans le solvant, les individus se tiennent éloignés du papier filtre imbibé de la composition odorante puis sont anesthésiés dans l'heure de test.

### Liste des références

1. Grosjean, Y., Grillet, M., Augustin, H., Ferveur, J.F. and Featherstone, D.E. A glial amino-acid transporter controls synapse strength and courtship in Drosophila. Nature Neuroscience 2008. Volume 11, pages 54-61. DOI: 10.1038/nn2019
2. Grosjean Y, Rytz R, Farine JP, Abuin L, Cortot J, Jefferis GS, Benton R. An olfactory receptor for food-derived odours promotes male courtship in Drosophila. Nature 2011. Volume 478(7368), pages 236-240. DOI: 10.1038/nature10428.
3. Greenspan & Ferveur. Courtship in Drosophila. Annu. Rev. Genet. 2000. Volume 34, pages 205-232. DOI: 10.1146/annurev.genet.34.1.205
4. Revadi S, Lebreton S, Witzgall P, Anfora G, Dekker T and Becher PG. Sexual Behavior of Drosophila suzukii. Insects 2015. Volume 6(1), pages 183-196 (2015). DOI: 10.3390/insects6010183.
5. Remington Pharmaceutical Sciences, seizième édition, E. W. Martin (Mack Publishing Co., Easton, Pa., 1980)

## Revendications

1. Utilisation d'au moins un acide gras choisi dans le groupe comprenant l'acide propionique, l'acide butyrique et/ou un dérivé de ceux-ci comme principe actif répulsif et/ou pour contrôler la reproduction de brachycères.

2. Utilisation selon la revendication 1 dans laquelle les brachycères sont choisis dans le groupe comprenant les insectes de la famille des muscidés, des oestridés, des hippoboscidés, des nyctéribiides, des syrphides, des tachinides, des thaumatoxénéiidés, des thermitoxénéiidés.

3. Utilisation selon la revendication 1 ou 2 dans laquelle les Brachycères sont choisis dans le groupe comprenant les insectes du genre musca, drosophila, glossina, calliphora, auchmeromyia, lucilia, sarcophaga, stomoxys.

4. Utilisation selon l'une quelconque des revendications 1 à 3 dans laquelle les brachycères sont choisis dans le groupe comprenant *Drosophila suzukii, Drosophila melanogaster.*

5. Utilisation selon l'une quelconque des revendications 1 à 4 dans laquelle le dérivé de l'acide propionique est choisi dans le groupe comprenant un ester, une amine ou un mélange de ceux-ci.

6. Utilisation selon l'une quelconque des revendications 1 à 5 dans laquelle l'acide propionique et/ou dérivé de celui-ci est à une concentration de 0,1% à 30%.

7. Utilisation selon l'une quelconque des revendications 1 à 6 dans laquelle le dérivé de l'acide butyrique est un ester d'acide butyrique.

8. Utilisation selon l'une quelconque des revendications 1 à 7 dans laquelle l'acide butyrique et/ou dérivé de celui-ci est à une concentration de 0,1% à 2%.

9. Utilisation selon l'une quelconque des revendications 1 à 8 dans laquelle l'acide propionique, l'acide butyrique et/ou un dérivé de ceux-ci sont dans une composition comprenant en outre un support acceptable.

10. Utilisation selon la revendication 9 dans laquelle le support acceptable est choisi parmi une huile minérale, une huile végétale.

11. Utilisation selon la revendication 10 dans laquelle l'huile minérale est l'huile de paraffine.

12. Utilisation selon l'une quelconque des revendications 1 à 11 dans laquelle l'acide gras défini dans les revendications 1 à 8 ou la composition définie dans les revendications 9 à 11 est appliquée au moyen d'un diffuseur, d'un spray, d'un pulvérisateur, d'un support imprégné.

13. Utilisation selon l'une quelconque des revendications 1 à 11 dans laquelle l'acide gras défini dans les revendications 1 à 8 ou la composition définie dans les revendications 9 à 11 est présente dans une trousse, la trousse comprenant en outre un support comprenant des instructions d'utilisations.

14. Procédé de protection d'au moins une surface biotique et/ou abiotique vis-à-vis de brachycères comprenant l'application sur ladite surface d'au moins un acide gras tel que défini dans les revendications 1 à 8 ou composition telle que définie dans les revendications 9 à 11.
